# EUROPEAN PATENT SPECIFICATION

(11) **EP 0 714 879 B1**
(45) Date of publication and mention of the grant of the patent: **04.08.1999**
(21) Application number: 95118432.4
(22) Date of filing: 23.11.1995
(51) Int. Cl.: C07C 43/225, C07C 43/23, C07C 59/70, C07C 235/06, C07C 235/16, C07C 235/20, A61K 49/04, C07C 235/48

(54) **Biphenyl iodinated derivatives and their diagnostic use**
Jodierte Biphenylderivate und ihre diagnostische Verwendung
Dérivés biphényles iodés et leur utilisation diagnostique

(30) Priority: 01.12.1994 IT MI942433
(43) Date of publication of application: 05.06.1996
(73) Proprietor: BRACCO S.p.A., 20134 Milano (IT); DIBRA S.p.A., 20122 Milano (IT)
(72) Inventor: Uggeri, Fulvio, I-20134 Milano (IT); Anelli, Pier Lucio, I-20134 Milano (IT); Brocchetta, Marino, I-20134 Milano (IT); Visigalli, Massimo, I-20134 Milano (IT)
(74) Representative: Minoja, Fabrizio, Dr.

(56) References cited:
- EP-A- 0 501 875
- EP-A- 0 609 586
- EP-A- 0 614 670
- WO-A-94/04488
- WO-A-95/15307
- DE-B- 1 179 336
- DE-C- 929 268

## Description

This invention refers to new contrast media particularly useful for X-ray diagnostic investigations of human and animal body and comprises new compounds of formula (I) and (II): 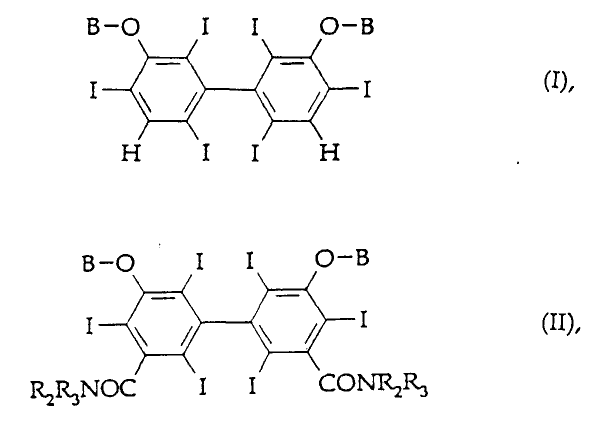 wherein
- B: is H or (C₁-C₆) hydroxyalkyl residue, with 1-5 -OH groups, or a polyoxaalkyl group of formula 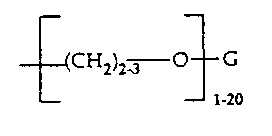 wherein
G is H, -CH₃ or -CH₂-CH₃,
   or
B is one of the groups of formula 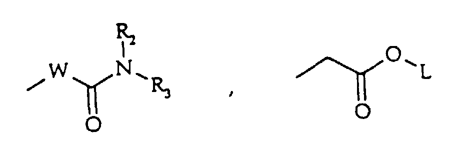 wherein
W is a -(CH₂)₀₋₅-CH(R₁)- group,
R₁ is H or a CH₃ group,
R₂ and R₃ equal or different among them, are H or a (C₁-C₆) alkyl group or a (C₁-C₆) hydroxyalkyl residue with 1-5 -OH groups, or a (C₂-C₆) alkoxyalkyl residue, or are a polyoxaalkyl group as previously defined, or R₂ and R₃, together, form a (C₃-C₆) chain, which can be substituted or not by - O-, -S-, -NH-, -N(CH₃)- groups, and
- L: is H or a (C₁-C₆) alkyl residue, or a (C₁-C₆) hydroxyalkyl residue, with 1-5 -OH groups.

Should the compounds of formula (I) include one or more phenolic or free acid functions, this invention comprise also the salts of said phenolic compounds with physiologically tolerable organic bases selected from primary, secondary and tertiary amines, or basic amino acids or inorganic bases whose cations are sodium, potassium, magnesium, calcium, or their mixtures.

Current X-ray contrast media preferably contain, as opacifying molecules, polyiodinated neutral aromatic compounds (see for instance D.P. Swanson et al. "Pharmaceuticals in Medical Imaging", 1990, Mac Millan Publ. Company).

These products comprise at least a benzene nucleus in which three hydrogen atoms are substituted with three iodine atoms. A good X-ray absorption requires a high iodine concentration and consequently said contrast agents are used in highly concentrated solutions, which can cause undesirable side effects (pain, high temperature, nausea, low pressure and vasal damages) due to hypertonicity and a viscosity higher than blood.

The products of this invention are new iodinated biphenyl derivatives which are characterized by the presence of 6 iodine atoms on the aromatic nuclei.

The derivatives of this invention have never been disclosed nor previously suggested. For instance, patent application EP-A-501875 (Guerbet) claims a formula like: 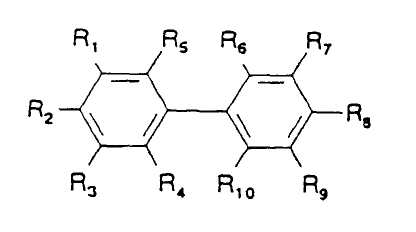 wherein R₁-R₁₀ can be an iodine atom or a group having the following formulae:

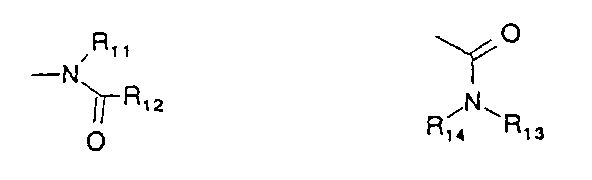

Nevertheless said formula does not comprise compounds in which there is one or more oxygenated function directly bound to aromatic rings, nor comprises the cases in which R₁-R₁₀ are hydrogen atoms; moreover EP-A-501875 does not claim nor specifically describes the compounds of this invention.

In the patent application WO 95/15307 (Bracco) are described compounds of the following formula:

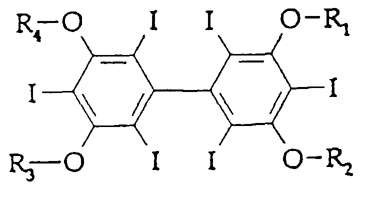

Said general formula comprise only compounds with oxygenated groups bound to the non-iodinated positions.

The compounds of this invention can be obtained according to known and commonly used synthetic ways for the preparation of iodinated contrast agents.

These compounds of the invention are generally characterized by high solubility, low viscosity and osmolality, as well as high intravenous, intracisternal and intracerebral tolerability.

Also, in view of their diagnostic use, they can be bound to or encapsulated in biomolecules or macromolecules which are aimed at selectively concentrating in the organ or the tissue under examination. Organ selectivity can be achieved, for instance, thanks to the encapsulation of said compounds into liposomes.

The compounds of this invention have a wide range of applications, since they can be used for intravasal, (for instance intravenous, intraarterial, intracoronaric, intraventricular administration and so on), intrathecal, intraperitoneal, intralymphatic, intracavital and intraparenchymal administration. Both soluble and less soluble compounds are suitable for oral or entheral administration, and therefore, specifically for the gastrointestinal tract imaging (GI). For parenteral administration they can be preferentially formulated as sterile aqueous solutions or suspensions, whose pH can range from 6.0 and 8.5, even thanks to the possible use of physiologically tolerable basic buffering agents (for instance tris[hydroxymethyl]aminomethane or TRIS).

These formulations can also be lyophilized and reconstituted just before the use. For GI use or for injection into body cavities, these agents can be formulated as a solution or suspension containing suitable additives, for example, to control viscosity.

For oral administration they can be formulated according to preparation methods routinely used in the pharmaceutical technique: excipients, such as sweeteners or flavouring agents, can be equally added according to known pharmaceutical formulation techniques.

As various changes could be made in the above compositions and methods without departing from the scope of the invention, it is intended that all matter contained in the above description shall be interpreted as illustrative.

### EXAMPLE 1

### 3,3'-bis(3,6,9,12,15,18,21-heptaoxadocos-1-yloxy)-2,2',4,4',6,6'-hexaiodo-1,1'-biphenyl

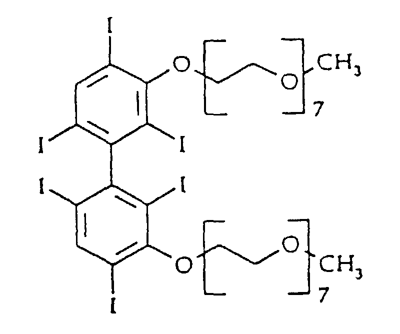

A) 2,2',4,4',6,6'-hexaiodo-1,1'-biphenyl-3,3'-diol
To a solution of 13.4 g (72 mmol) of 1,1'-biphenyl-3,3'-diol (CAS RN 612-76-0) in 900 mL of a H₂O : CH₃CN = 1 : 1 mixture at room temperature, 111 g (684 mmol) of a ICl solution (45% I; 16% HCl) in 93 mL of 37% HCl are added. After 3 h at 60°C, MeOH is added to obtain a solution. The solution is stirred at 60°C for 20 days while 2470 mL of MeOH are added periodically in portions of about 225 mL. The mixture is then allowed to cool to room temperature and concentrated causing the precipitation of crude product. 500 mL of water are added and the suspension is kept at 5°C over night and then filtered. The residue is washed with water and dried in vacuo at 20°C. The crude is dissolved in AcOEt and washed with a 5% sodium thiosulfate solution and with water, dried and evaporated at 40°C, to give 86.3 g (92 mmol) of the desired product.
Yield: 88.8% mp (DSC): 269.7°C (dec.)
HPLC: 97.9% (area %)
Stationary phase: Nucleosil C18, length 250 mm, i.d. 4mm, 5µm
Eluent A: 10% acetonitrile/89.9% water/0.1% H₃PO₄(85%ic)
Eluent B: 90% acetonitrile/9.9% water/0.1% H₃PO₄(85%ic)
Gradient timetable:

| min | %A | %B |
|---|---|---|
| 0 | 70% | 30% |
| 20 | 5% | 95% |
| 25 | 5% | 95% |
| 10 | posttime | |

Flow: 1 mL/min
Temperature: 20-25°C
Concentration: 1 mg/mL
Detection (UV): 280 nm

| Element. analysis | C | H | I | O |
|---|---|---|---|---|
| % calc.: | 15.31 | 0.43 | 80.86 | 3.40% |
| % found: | 15.83 | 0.49 | 79.40 | 4.03 |

¹H-NMR, ¹³C-NMR, IR and MS spectra are consistent with the structure.

B) 4-nitrobenzenesulfonic acid 3,6,9,12,15,18,21-heptaoxadocos-1-yl ester
6.7 g (60 mmol) of TEA are dropwise added, in 10 min, to a solution of 20.4 g (60 mmol) of 4-nitrobenzenesulfonyl chloride (commercial product) and of 20.4 g (60 mmol) of 3,6,9,12,15,18,21-heptaoxadocosan-1-ol (according to Liebigs Ann. Chem. 1980, 858-862) in 60 mL of AcOEt. After 2 h at 20°C the precipitated TEA hydrochloride is filtered off and washed with 10 mL of AcOEt for three times. The filtrates are collected, washed with 30 mL of H₂O, 20 mL of 2N HCl and then with H₂O to reach a neutral pH. The organic phase is evaporated to dryness, to give a thick oil which is directly used in the successive step without further purification. 25 g (47 mmol) of 4-nitrobenzenesulfonic-3,6,9,12,15,18,21-heptaoxadocos-1-yl acid ester are obtained.
- Yield:: 79 %

¹H-NMR, ¹³C-NMR, IR and MS spectra are consistent with the structure.

C) 3,3'-bis(3,6,9,12,15,18,21-heptaoxadocos-1-yloxy)-2,2',4,4',6,6'-hexaiodo-1,1'-biphenyl
To a solution of 15.06 g (16 mmol) of compound A) in 320 mL of DMA, 16 mL (32 mmol) of 2N MeONa in MeOH are added and after evaporation of MeOH, 25.23 g (48 mmol) of compound B) are added. The reaction mixture is heated at 70°C for 6h. The solution is evaporated under vacuum and the resulting oily residue is treated with CH₂Cl₂. The resulting solid is removed while the solution is washed with H₂O, dried and concentrated to dryness. The residue is treated with AcOEt to give 17.45 g (11 mmol) of the desired product.
- Yield:: 69%

¹H-NMR, ¹³C-NMR, IR and MS spectra are consistent with the structure.

### EXAMPLE 2

### 1,1'-[(2,2',4,4',6,6'-hexaiodo-1,1'-biphenyl)-3,3'-diylbis[oxy(1-oxo-2,1-ethanediyl)methylimino]]bis[1-deoxy-D-glucitol]

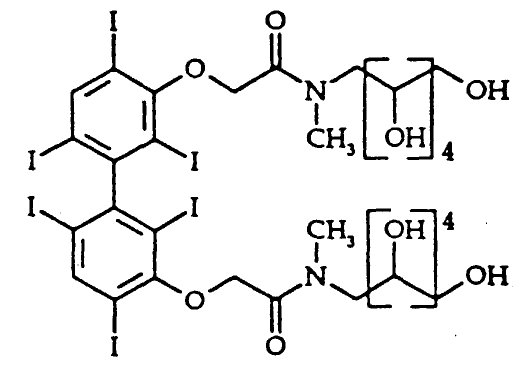

A) 2,2'-[(2,2',4,4',6,6'-hexaiodo-1,1-biphenyl)-3,3'-diylbis-(oxy)]bisacetic acid dimethyl ester
A 1.1 M solution of MeONa in MeOH (63 ml; 0.0693 mol) is dropped in 10 min into a solution of 28.2 g (0.03 mol) of 2,2',4,4',6,6'-hexaiodo-1,1'-biphenyl-3,3'-diol (prepared according to EXAMPLE 1) in DMF (240 ml) stirred at room temperature. After 5 min MeOH is distilled, methyl bromoacetate (11.2 g; 0.073 mol) is added and the solution is stirred at 45 °C for 4.5 h. The reaction mixture is filtered, then distilled and the residue is treated with H₂O. The resulting solid is filtered, washed with H₂O and stirred at 50 °C with MeOH for 30 min. After 15 h at room temperature the solid is filtered, washed with MeOH and dried to give the desired product (30 g; 0.0276 mol).
Yield: 92% mp: 174-7 °C
HPLC : 97.1% (area %)
Stationary phase: Merck Lichrospher 100 RP-18 5 µm; 250 x 4 mm;
Temperature: 35°C;
Mobile phase: gradient elution;
Eluent A = 0.017 M H₃PO₄ in water;
Eluent B = CH₃CN;
Gradient timetable:

| min | % A | % B |
|---|---|---|
| 0 | 40 | 60 |
| 15 | 5 | 95 |
| 30 | 5 | 95 |

Flow rate: 1 ml min⁻¹;
Detection (UV): 225 nm;
Injection: 10 µl;
Concentration: 1 mg ml⁻¹;

| Element. analysis | C | H | I | |
|---|---|---|---|---|
| % calc.: | 19.91 | 1.11 | 70.13% | |
| % found: | 19.95 | 1.15 | 70.11 | H₂O <0.1 |

¹H-NMR, ¹³C-NMR, IR and MS spectra are consistent with the structure.

B) 1,1'-[(2,2',4,4',6,6'-hexaiodo-1,1'-biphenyl)-3,3'-diylbis[oxy(1-oxo-2,1-ethanediyl)methylimino]]bis[1-deoxy-D-glucitol]
To a solution of 9.6 g (8.84 mmol) of compound A) in 130 mL of MeOH heated at 50°C, 11.22 g (57.46 mmol) of N-methylglucamine are added by heating at 50°C for about 5h. The reaction mixture is reduced to a third of the volume and cooled to room temperature. The crystallized solid is filtered, washed and dried; the crude is purified by crystallization from MeOH to give 7.73 g (5.57 mmol) of the desired product.
- Yield:: 63%

¹H-NMR, ¹³C-NMR, IR and MS spectra are consistent with the structure.

### EXAMPLE 3

### 2,2'-[(2,2',4,4',6,6'-hexaiodo-1,1'-biphenyl)-3,3'-diylbis(oxy)]bis[N-(2,3-dihydroxypropyl)acetamide]

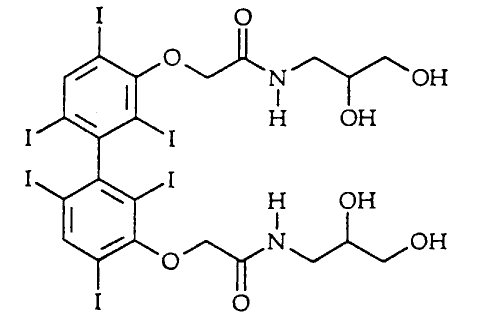

Following the procedure of EXAMPLE 2, 8.7 g (8 mmol) of 2,2'-[(2,2',4,4',6,6'-hexaiodo-1,1-biphenyl)-3,3'-diylbis-(oxy)]bisacetic acid dimethyl ester are reacted with 4.74 g (52 mmol) of isoserinol in 120 mL of MeOH, and boiled over 20 h. 6.5 g (5.4 mmol) of the desired product are obtained.
- Yield:: 68%

¹H-NMR, ¹³C-NMR, IR and MS spectra are consistent with the structure.

### EXAMPLE 4

### 2,2'-[(2,2',4,4',6,6'-hexaiodo-1,1'-biphenyl)-3,3'-diylbis(oxy)]bis[N-methylacetamide]

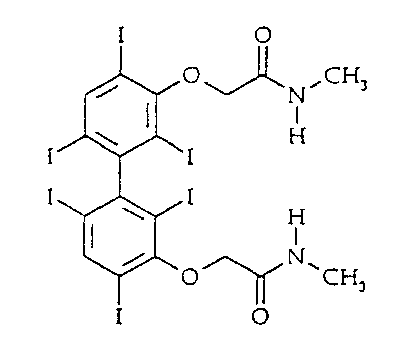

Following the procedure of EXAMPLE 2, 10.75 g (9.9 mmol) of 2,2'-[(2,2',4,4',6,6'-hexaiodo-1,1-biphenyl)-3,3'-diylbis-(oxy)]bisacetic acid dimethyl ester are reacted with 5 g (64.4 mmol) of 40%aq. MeNH₂ in 150 mL of MeOH. 8.78 g (8.1 mmol) of the desired product are obtained.
- Yield:: 82%

¹H-NMR, ¹³C-NMR, IR and MS spectra are consistent with the structure.

### EXAMPLE 5

### 2,2'-[(2,2',4,4',6,6'-hexaiodo-1,1'-biphenyl)-3,3'-diylbis(oxy)]bisacetic acid

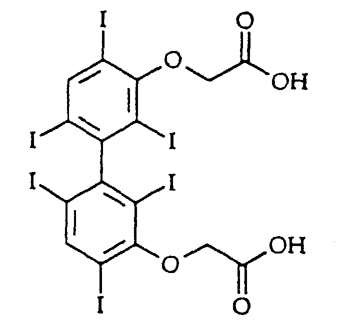

A 1M solution of NaOH (46 ml; 0.046 mol) is dropped in 9 h into a solution of 2,2'-[[2,2',4,4',6,6'-hexaiodo-1,1'-biphenyl]-3,3'-diylbis(oxy)]bis acetic acid methyl ester (prepared according to EXAMPLE 2) (24.9 g; 0.023 mol) in 1,4-dioxane (300 ml)/H₂O (75 ml) stirred at 50 °C, maintaining the pH between 11.5 and 10.5. The pH is then adjusted to 8.5 and the reaction mixture is evaporated to dryness; the residue is dissolved in H₂O (300 ml), decolorized (Acticarbone^{R} 2S), filtered and poured in 0.28M HCl (210 ml; 0.059 mol) under vigorous stirring. After 15 h at room temperature the solid is filtered, washed with H₂O and dried to give the desired product (23.1 g; 0.0218 mol).
Yield: 95% mp: 162-6 °C
HPLC : 98.8% (area %)
Stationary phase: Merck Lichrospher 100 RP-18 5 µm; 250 x 4 mm;
Temperature: 35°C;
Mobile phase: gradient elution;
Eluent A = 0.017 M H₃PO₄ in water;
Eluent B = CH₃CN;
Gradient timetable:

| min | % A | % B |
|---|---|---|
| 0 | 40 | 60 |
| 15 | 5 | 95 |
| 30 | 5 | 95 |

Flow rate: 1 ml min⁻¹;
Detection (UV): 225 nm;
Injection: 10 µl;
Concentration: 1 mg ml⁻¹;

| Element. analysis | C | H | I | |
|---|---|---|---|---|
| % calc.: | 18.17 | 0.76 | 71.99 | |
| % found: | 18.14 | 0.847 | 1.97 | H₂O 0.28 |

¹H-NMR, ¹³C-NMR, IR and MS spectra are consistent with the structure.

### EXAMPLE 6

### 2,2'-[(2,2',4,4',6,6'-hexaiodo-1,1'-biphenyl)-3-3'-diylbis(oxy)]acetamide

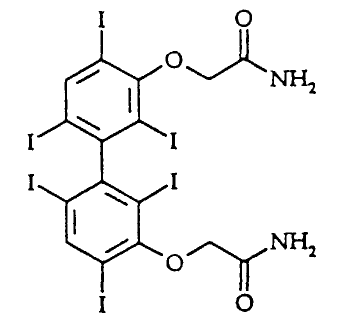

9.85 g of 2,2'-[(2,2',4,4',6,6'-hexaiodo-1,1'-biphenyl)-3,3'-diol disodium salt (prepared according to EXAMPLE 2) (0.01 mol) are dissolved in 50 mL of DMF. 2.9 g of 2-bromoacetamide (0.021 mol) are added to the solution and the whole is heated at 45-50°C for 30 h. Then the mixture is cooled to r.t. and added to a solution of 500 mL of water and 50 mL of NH₄OH 25%. After 2h under stirring and one night without, the precipitate is filtered, washed with water, obtaining a crude which is purified by stirring with CHCl₃/MeOH/NH₄OH 25% = 5/3/1, filtration and drying to give 7 g of the desired product (0.007 mol).
Yield: 66%
HPLC: 98.2% (area %)
Stationary phase: E. Merck Lichrospher 100 RP-18 5µm, 250 x 4 mm
Temperature: 35°C
Mobile phase: gradient elution
Eluent A = 0.017 M H₃PO₄ in water
Eluent B = CH₃CN
Gradient timetable:

| min | % A | % B |
|---|---|---|
| 0 | 85 | 15 |
| 20 | 50 | 50 |
| 30 | 25 | 75 |
| 45 | 25 | 75 |

Flow rate: 1 ml min⁻¹
Detection (UV): 225 nm, 245 nm
Injection: 10 µl
Concentration: 1 mg ml⁻¹;

| Element. analysis | C | H | I | N | |
|---|---|---|---|---|---|
| % calc.: | 20.22 | 1.52 | 67.45 | 3.72% | |
| % found: | 20.03 | 1.43 | 66.95 | 3.66 | H₂O 1.1 |

¹H-NMR, ¹³C-NMR, IR and MS spectra are consistent with the structure.

### EXAMPLE 7

### 2,2'-[[2,2',4,4',6,6'-hexaiodo-1,1'-biphenyl]-3,3'-diylbis(oxy)]bis [N-[2-(2-hydroxyethoxy)ethyl]acetamide]

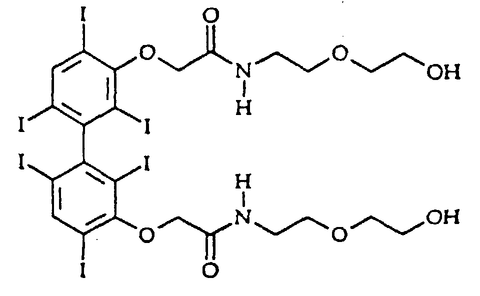

Quinoline (0.013 g; 0.1 mmol) is added to a suspension of 2,2'-[(2,2',4,4',6,6'-hexaiodo-1,1'-biphenyl)-3,3'-diylbis(oxy)]bisacetic acid (prepared according to EXAMPLE 5) (32.34 g; 0.031 mol) in SOCl₂ (147.5 g; 1.240 mol) then the mixture is heated at 70 °C for 2.5 h ; the solution is concentrated and after 1 h at room temperature the crystalline precipitate is filtered, washed with n-hexane and dried. The chloride thus obtained is dissolved with DMF and the solution quickly dropped into a solution of 2-(2-aminoethoxy)ethanol (12.6 g; 0.120 mol) in DMF (1000 ml) at room temperature; after 3 h the mixture is evaporated under reduced pressure (0.2 kPa) and the residue is dropped into H₂O. The precipitate is filtered, washed with water and dried; the crude is purified by flash chromatography (eluent CHCl₃/EtOH 96% = 95/5) to give the desired product (23.5 g; 0.019 mol).
Yield: 62 % m.p. : 197 - 200 °C
HPLC : 98.1 % (area %)
Stationary phase: E. Merck Lichrospher 100 RP-18 5µm, 250 x 4 mm
Temperature: 35°C
Mobile phase: gradient elution
Eluent A = 0.017 M H₃PO₄ in water
Eluent B = CH₃CN
Gradient timetable:

| min | % A | % B |
|---|---|---|
| 0 | 85 | 15 |
| 20 | 50 | 50 |
| 30 | 25 | 75 |
| 45 | 25 | 75 |

Flow rate: 1 ml min⁻¹
Detection (UV): 225 nm, 245 nm
Injection: 10 µl
Concentration: 1 mg ml⁻¹;

| Element. analysis | C | H | I | N | |
|---|---|---|---|---|---|
| % calc.: | 23.40 | 2.13 | 61.81 | 2.27 | |
| % found: | 23.30 | 2.11 | 61.55 | 2.25 | H₂O 0,52 |

TLC Stationary phase : Silica gel plates 60 F₂₅₄ (E. Merck art. 5715)
Eluent: 9 : 1 = CH₂Cl₂/MeOH
Detection: UV (254 nm) or exposition to UV light (254 nm) after treatment with soluble starch or 1% KMnO₄ in 1M NaOH Rf = 0.74
¹H-NMR, ¹³C-NMR, IR and MS spectra are consistent with the structure.

### EXAMPLE 8

### N,N'-bis[2-hydroxy-1-(hydroxymethyl)ethyl]-5,5'-dihydroxy-2,2',4,4',-6,6'-hexaiodo-1,1'-biphenyl-3,3'-dicarboxamide

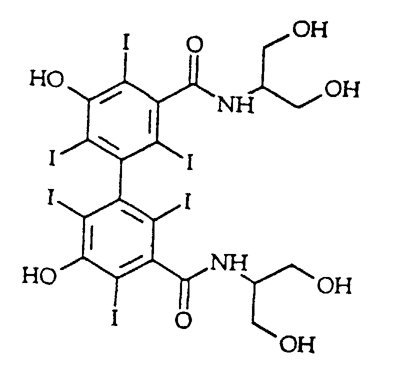

A) 5,5'-diamino-1,1'-biphenyl-3,3'-dicarboxylic acid dimethyl ester.
   A solution of 30 g (83.3 mmol) of 5,5'-dinitro-1,1'-biphenyl-3,3'-dicarboxylic acid dimethyl ester (prepared according to patent application WO 9404488) in 1.6 L of MeOH is hydrogenated in autoclave at 60°C in the presence of 42 g of 10% Pd/C under a pressure of 6 atm of H₂; after 6h the catalyst is filtered and the solution is concentrated to dryness to give 23.5 g (78.3 mmol) of the desired product.
   - Yield:: 94%

   ¹H-NMR, ¹³C-NMR, IR and MS spectra are consistent with the structure.
B) 5,5'-dihydroxy-1,1'-biphenyl-3,3'-dicarboxylic acid dimethyl ester
   20 g (66.6 mmol) of compound A) are suspended in 130 mL of H₂O and dissolved by addition of 157 g (400 mmol) of 25% H₂SO₄. To this solution, cooled at 0-5°C, a solution of 11.04 g (160 mmol) of NaNO₂ in 35 mL of H₂O is added in 15 min. After 15 min at 5°C, the reaction mixture is added dropwise, in 30 min, to a solution of 47.3 g (333 mmol) of Na₂SO₄ and 66.6 g (666 mmol) of H₂SO₄ 98% in 70 mL of H₂O and heated at 70°C. After 2h at this temperature, the resulting precipitate is filtered, washed with H₂O and dried. The crude is refluxed with MeOH/HCl_{(g)} and after evaporation of the solvent, the resulting solid is purified by crystallization from MeOH to give 15.42 g (51 mmol) of the desired product.
   - Yield:: 77%

   ¹H-NMR, ¹³C-NMR, IR and MS spectra are consistent with the structure.
C) N,N'-bis[2-hydroxy-1-(hydroxymethyl)ethyl]-5,5'-dihydroxy-1,1'-biphenyl-3,3'-dicarboxamide
   A mixture of 10.3 g (34 mmol) of compound B) and 24.78 g (272 mmol) of 2-amino-1,3-propandiol is heated under vacuum (100°C; 2.6 KPa) for about 3h up to complete development of MeOH. The molten mass is cooled to 50°C and dissolved in 100 mL of H₂O. The solution is percolated on a cation exchange resin Duolite^{R} C 20 MB and then on an anion exchange resin Duolite^{R} A 30 B, through elution with water; the neutral eluate is concentrated to dryness to give a crude which is crystallized from abs. EtOH to give 8.83 g (21 mmol) of the desired product.
   - Yield:: 62%

   ¹H-NMR, ¹³C-NMR, IR and MS spectra are consistent with the structure.
D) N,N'-bis[2-hydroxy-1-(hydroxymethyl)ethyl]-5,5'-dihydroxy-2,2',4,4',6,6'-hexaiodo-1,1'-biphenyl-3,3'-dicarboxamide
   To a solution of 8 g (19 mmol) of compound C) in 60 mL of H₂O at room temperature in about 3h, 32.15 g (114 mmol) of a solution of ICl (45% I; 16% HCl) and 57 mL (285 mmol) of 5N NaOH kept at pH 8 are added. The resulting solution is treated with NaHSO₃, filtered and adjusted to pH 2 with 37% HCl. The crude precipitate is filtered, washed with H₂O, dried and purified through dissolution in H₂O at pH 8 with 2N NaOH and further reprecipitation at pH 2 with 37% HCl. After filtration, washing with H₂O and drying, 17.64 g (15 mmol) of the desired product are obtained.
   - Yield:: 79%

   ¹H-NMR, ¹³C-NMR, IR and MS spectra are consistent with the structure.

### EXAMPLE 9

### N,N'-bis[2-hydroxy-1-(hydroxymethyl)ethyl]-5,5'-bis(3,6,9,12,15,18,21-heptaoxadocos-1-yloxy)-2,2',4,4',6,6'-hexaiodo-1,1'-biphenyl-3,3'-dicarboxamide

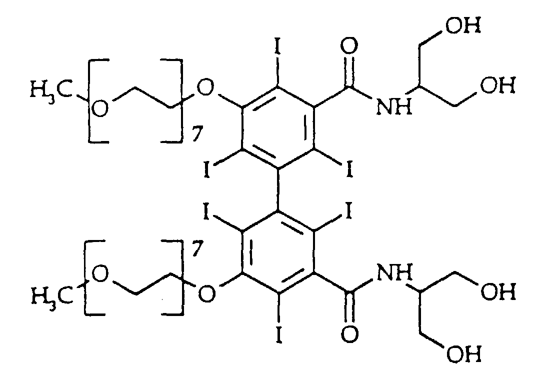

To a solution of 8.3 g (6.8 mmol) of N,N'-bis[2-hydroxy-1-(hydroxymethyl)ethyl]-5,5'-dihydroxy-2,2',4,4',6,6'-hexaiodo-1,1'-biphenyl-3,3'-dicarboxamide disodium salt [prepared through concentration to dryness of a solution of 8 g (6.8 mmol) of N,N'-bis[2-hydroxy-1-(hydroxymethyl)ethyl]-5,5'-dihydroxy-2,2',4,4',6,6'-hexaiodo-1,1'-biphenyl-3,3'-dicarboxamide, described in EXAMPLE 6, and 13.6 mL (13.6 mmol) of 1N NaOH in 50 mL of H₂O] in 170 mL of DMA, 11.79 g (22.44 mmol) of 4-nitrobenzenesulfonic acid 3,6,9,12,15,18,21-heptaoxadocos-1-yl ester (prepared according to EXAMPLE 1) are added and the mixture is heated at 70°C for 7h. The mixture is then evaporated under vacuum and the oily residue is treated with CH₂Cl₂. The resulting precipitate is filtered and removed while the organic phase is washed with H₂O, dried and evaporated to dryness. The resulting residue is boiled over with AcOEt and after cooling at 25°C, filtration and drying, 8.89 g (4.9 mmol) of the desired product are obtained.
- Yield:: 72%

¹H-NMR, ¹³C-NMR, IR and MS spectra are consistent with the structure.

### EXAMPLE 10

### 2,2'-[[5,5'-bis[[[2-hydroxy-1-(hydroxymethyl)ethyl]amino]carbonyl]-2,2',4,4',6,6'-hexaiodo-1,1'-biphenyl]-3,3'-diylbis(oxy)]bisacetic acid dimethyl ester

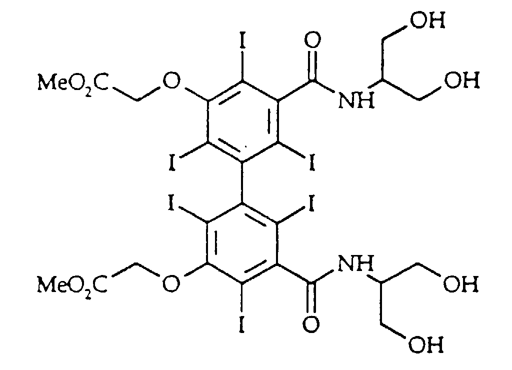

To a solution of 9.03 g (7.4 mmol) of N,N'-Bis[2-hydroxy-1-(hydroxymethyl)ethyl]-5,5'-dihydroxy-2,2',4,4',6,6'-hexaiodo-1,1'-biphenyl-3,3'-dicarboxamide disodium salt (prepared according to EXAMPLE 9) in 200 mL of DMA, 3.81 g (24.42 mmol) of BrCH₂CO₂Me are added. After 4h at 50°C the solvent is evaporated under reduced pressure and the oily residue is treated with H₂O, to give a precipitate which is filtered, washed with H₂O and dried. The resulting crude is crystallized from MeOH to give 6.2 g (4.7 mmol) of the desired product.
- Yield:: 64%

¹H-NMR, ¹³C-NMR, IR and MS spectra are consistent with the structure.

### EXAMPLE 11

### N,N'-bis[2-hydroxy-1-(hydroxymethyl)ethyl]-5,5'-bis[[2-[2-(hydroxyethoxy)ethyl]methylamino]-2-oxoethoxy]-2,2',4,4',6,6'-hexaiodo-1,1'-biphenyl-3,3'-dicarboxamide

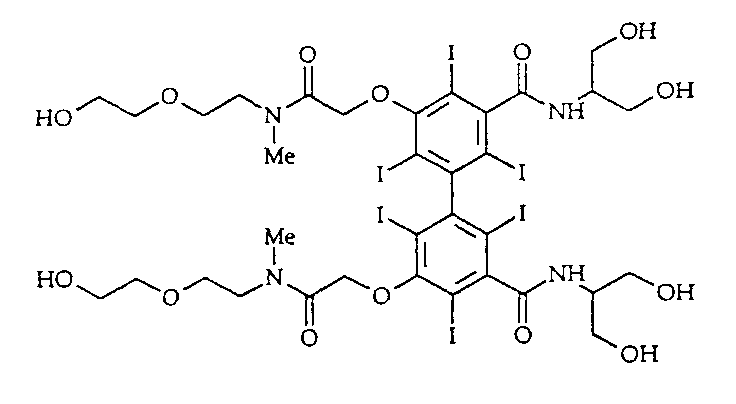

To a solution of 5.81 g (4.4 mmol) of 2,2'-[[5,5'-bis[[[2-hydroxy-1-(hydroxymethyl)ethyl]amino]carbonyl]-2,2',4,4',6,6'-hexaiodo-1,1'-biphenyl]-3,3'-diylbis(oxy)]bisacetic acid dimethyl ester (prepared according to EXAMPLE 10) in 70 mL of MeOH, heated at 50°C, 3.4 g (28.6 mmol) of 2-[2-(methylamino)ethoxy]ethanol (CAS RN 85475-01-0) are added. Then the solution is boiled for 4h; the reaction mixture is concentrated to dryness; the residue is dissolved in H₂O and the solution is percolated on a cation exchange resin Duolite^{R} C 20 MB and then on a on an anion exchange resin Duolite^{R} A 30 B. The neutral eluate is concentrated to dryness and the residue is crystallized from EtOH to give 5 g (3.4 mmol) of the desired product.
- Yield:: 77%

¹H-NMR, ¹³C-NMR, IR and MS spectra are consistent with the structure.

### EXAMPLE 12

### 2,2'-[[5,5'-bis[[[2-hydroxy-1-(hydroxymethyl)ethyl]amino]carbonyl]-2,2',4,4',6,6'-hexaiodo-1,1'-biphenyl]-3,3'-diylbis(oxy)]bisacetic acid

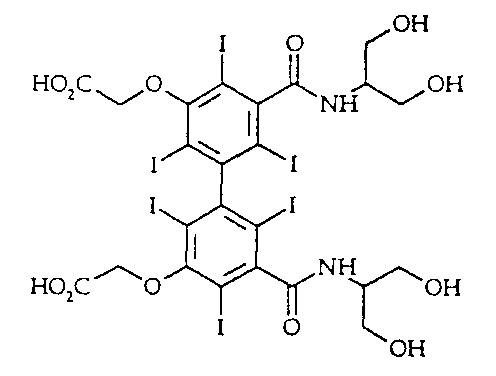

To a suspension of 3.96 g (3 mmol) of 2,2'-[[5,5'-bis[[[2-hydroxy-1-(hydroxymethyl)ethyl]amino]carbonyl]-2,2',4,4',6,6'-hexaiodo-1,1'-biphenyl]-3,3'-diylbis(oxy)]bisacetic acid dimethyl ester (prepared according to EXAMPLE 10) in 50 mL of H₂O 7 mL of 1N NaOH (7 mmol) are added at room temperature obtaining a gradual dissolution; after 2h the solution is filtered and adjusted to pH 1.5 with 37% HCl thus obtaining the precipitation of 3.62 g (2.8 mmol) of the desired product.
- Yield:: 93%

¹H-NMR, ¹³C-NMR, IR and MS spectra are consistent with the structure.

## Claims

1. Compounds of formula (I) and their salts 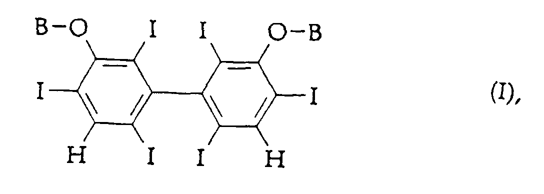 wherein
B is H or (C₁-C₆) hydroxyalkyl residue, with 1-5 -OH groups, or a polyoxaalkyl group of formula 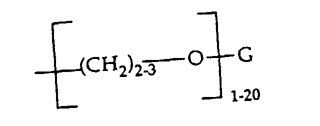 wherein
G is H, -CH₃ or -CH₂-CH₃,
or
B is one of the groups of formula 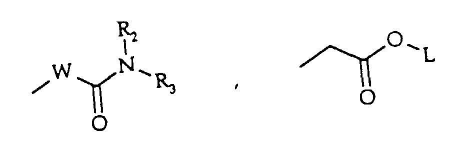 wherein
W is a -(CH₂)₀₋₅-CH(R₁)- group,
R₁ is H or a CH₃ group,
R₂ and R₃ equal or different among them, are H or a (C₁-C₆) alkyl group or a (C₁-C₆) hydroxyalkyl residue with 1-5 -OH groups, or a (C₂-C₆) alkoxyalkyl residue, or are a polyoxaalkyl group as previously defined, or R₂ and R₃, together, form a (C₃-C₆) chain, which can be substituted or not by - O-, -S-, -NH-, -N(CH₃)- groups, and
L is H or a (C₁-C₆) alkyl residue, or a (C₁-C₆) hydroxyalkyl residue, with 1-5 -OH groups.

2. Compounds of formula (II) and their salts 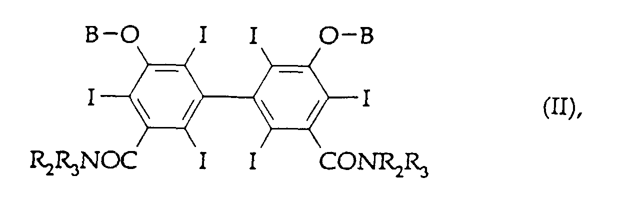 wherein R₂ and R₃ are as previously defined in claim 1.

3. A compound according to claims 1-2, selected from the group constituted by:
- 2,2',4,4',6,6'-hexaiodo-1,1'-biphenyl-3,3'-diol;
- 3,3'-bis(3,6,9,12,15,18,21-heptaoxadocos-1-yloxy)-2,2',4,4',6,6'-hexaiodo-1,1'-biphenyl;
- 2,2'-[(2,2',4,4',6,6'-hexaiodo-1,1-biphenyl)-3,3'-diylbis-(oxy)]bisacetic acid dimethyl ester;
- 1,1'-[(2,2',4,4',6,6'-hexaiodo-1,1'-biphenyl)-3,3'-diylbis[oxy(1-oxo-2,1-ethanediyl)methylimino]]bis[1-deoxy-D-glucitol];
- 2,2'-[(2,2',4,4',6,6'-hexaiodo-1,1'-biphenyl)-3,3'-diylbis(oxy)]bis[N-(2,3-dihydroxypropyl)acetamide];
- 2,2'-[(2,2',4,4',6,6'-hexaiodo-1,1'-biphenyl)-3,3'-diylbis(oxy)]bis[N-methylacetamide];
- 2,2'-[(2,2',4,4',6,6'-hexaiodo-1,1'-biphenyl)-3,3'-diylbis-(oxy)]bisacetic acid;
- 2,2'-[(2,2',4,4',6,6'-hexaiodo-1,1'-biphenyl)-3-3'-diylbis(oxy)]acetamide;
- 2,2'-[[2,2',4,4',6,6'-hexaiodo-1,1'-biphenyl]-3,3'-diylbis(oxy)]bis[N-[2-(2-hydroxyethoxy)ethyl]acetamide];
- 2[3-[3-[(hydroxyacetyl)[2-(2-hydroxyethoxy)ethyl]-amino]-2,4,6-triiodophenyl]-2,4,6-triiodophenoxy]-N-[2-(2-hydroxyethoxy)ethyl]acetamide;

4. Contrast media composition useful for X-ray diagnosis of human and animal body comprising at least one of the compound of claim 1 or 3 or the salts thereof.

5. Use of at least one of the compounds according to claims 1-3 for the preparation of X-ray diagnostic formulations.

6. Use of formulations according to claim 5, to obtain images of organs or tissues of human and animal body in X-ray diagnostic imaging.

## Patentansprüche

1. Verbindungen der Formel (I) und ihre Salze 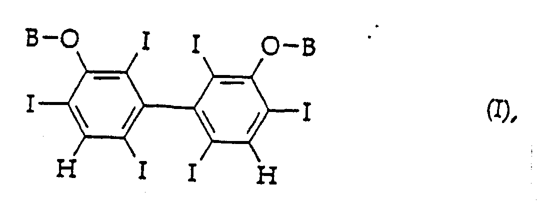 worin
B die Bedeutung von H oder einem (C₁-C₆)Hydroxyalkylrest mit 1 - 5 -OH-Gruppen oder einer Polyoxyalkylgruppe der Formel 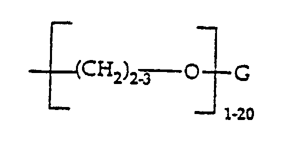 hat, worin
G die Bedeutung von H, -CH₃ oder -CH₂-CH₃ hat,
oder
B eine der Gruppen der Formel 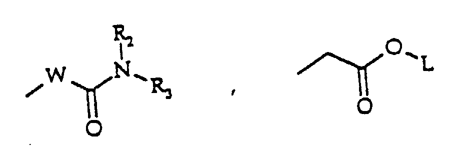 ist, worin
W eine -(CH₂)₀₋₅-CH(R₁)-Gruppe ist,
R₁ die Bedeutung hat von H oder einer CH₃-Gruppe,
R₂ und R₃ die gleich oder verschieden voneinander sein können, H oder eine (C₁-C₆)Alkylgruppe oder ein (C₁-C₆)Hydroxyalkylrest mit 1-5 -OH-Gruppen oder ein (C₂-C₆)Alkoxyalkylrest oder eine Polyoxyalkylgruppe, wie vorstehend definiert, sind, oder R₂ und R₃ miteinander eine (C₃-C₆)-Kette bilden, die substituiert oder unsubstituiert sein kann durch -O-, -S-, -NH-, -N(CH₃)-Gruppen und
L die Bedeutung hat von H oder einem (C₁-C₆)Alkylrest, oder einem (C₁-C₆)Hydroxyalkylrest mit 1-5 -OH-Gruppen.

2. Verbindungen der Formel (II) und ihre Salze 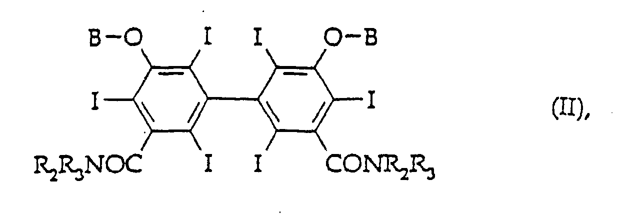 worin R₂ und R₃ wie vorstehend in Anspruch 1 definiert sind.

3. Verbindung nach den Ansprüchen 1-2, ausgewählt, aus der Gruppe von
- 2,2',4,4',6,6'-Hexaiod-1,1'-biphenyl-3,3'-diol;
- 3,3'-Bis(3,6,9,12,15,18,21-heptaoxadocos-1-yloxy)-2,2',4,4',6,6'-hexaiod-1,1'-biphenyl;
- 2,2'-[(2,2',4,4',6,6'-Hexaiod-1,1-biphenyl)-3,3'-diylbis-(oxy)]bisessigsäuredimethylester;
- 1,1'-[(2,2',4,4',6,6'-Hexaiod-1,1'-biphenyl)-3,3'-diylbis[oxy(1-oxo-2,1-ethandiyl)methylimino]]bis[1-deoxy-D-glucitol];
- 2,2'-[(2,2',4,4',6,6'-Hexaiod-1,1'-biphenyl)-3,3'-diylbis(oxy)]bis[N-(2,3-dihydroxypropyl)acetamid];
- 2,2'-[(2,2',4,4',6,6'-Hexaiod-1,1'-biphenyl)-3,3'-diylbis(oxy)]bis[N-methylacetamid];
- 2,2'-[(2,2',4,4',6,6'-Hexaiod-1,1'-biphenyl)-3,3'-diylbis(oxy)]bisessigsäure;
- 2,2'-[(2,2',4,4',6,6'-Hexaiod-1,1'-biphenyl)-3,3'-diylbis(oxy)]acetamid;
- 2,2'-[(2,2',4,4',6,6'-Hexaiod-1,1'-biphenyl]-3,3'-dilylbis(oxy)]bis[N-[2-(2-hydroxyethoxy)ethyl]acetamid];
- 2[3-[3-[(-Hydroxyacetyl)[2-(2-hydroxyethoxy)ethyl]amino]-2,4,6-triiodphenyl]-2,4,6-triiodphenoxy]-N-[2-(2-hydroxyethoxy)ethyl]acetamid.

4. Kontrastmittelzusammensetzung, geeignet zur Röntgenstrahlendiagnose des menschlichen und tierischen Körpers, umfassend mindestens eine der Verbindungen nach Anspruch 1 oder 3 oder deren Salze.

5. Verwendung mindestens einer der Verbindungen nach den Ansprüchen 1-3 zur Herstellung von Formulierungen zur Röntgenstrahlendiagnose.

6. Verwendung von Formulierungen nach Anspruch 5 zur Erzielung von Abbildungen von Organen oder Geweben des menschlichen oder tierischen Körpers bei der Röntgenstrahlen-Bilddarstellungs-Diagnose.

## Revendications

1. Composés de formule (I) et leurs sels 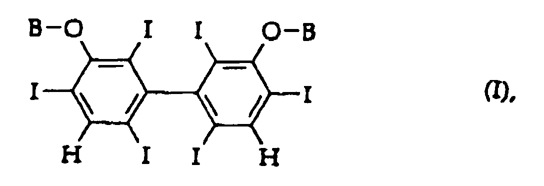 dans laquelle
B est H ou un reste hydroxyalkyle en C₁ à C₆, avec 1 a 5 groupes -OH, ou un groupe polyoxaalkyle de formule 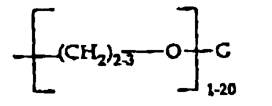 dans laquelle
G est H, -CH₃ ou -CH₂-CH₃,
ou B est l'un des groupes de formule 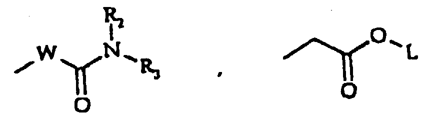 dans lesquelles
W est un groupe -(CH₂)₀₋₅-CH(R₁)-,
R₁ est H ou un groupe CH₃,
R₂ et R₃, identiques ou différents entre eux, sont H ou un groupe alkyle en C₁ à C₆ ou un reste hydroxyalkyle en C₁ à C₆ avec 1 à 5 groupes -OH, ou un reste alcoxyalkyle en C₂ à C₆,ou un groupe polyoxaalkyle tel que défini précédemment, ou bien R₂ et R₃, ensemble, forment une chaîne en C₃ à C₆, qui peut être substituée ou non par O-, -S-, -NH-, -N(CH₃)-, et
L est H ou un reste alkyle en C₁ à C₆, ou un reste hydroxyalkyle en C₁ à C₆ avec 1 à 5 groupes -OH.

2. Composés selon la formule (II) et leurs sels 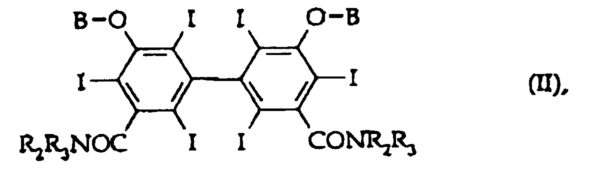 dans laquelle R₂ et R₃ sont tels que définis précédemment dans la revendication 1.

3. Composé selon les revendications 1-2, choisi dans le groupe constitué par :
- le 2,2',4,4',6,6'-hexaiodo-1,1'-biphényl-3,3'-diol ;
- le 3,3'-bis(3,6,9,12,15,18,21-heptaoxadocos-1-yloxy)-2,2',4,4',6,6'-hexaiodo-1,1'-biphényle ;
- l'ester diméthylique de l'acide 2,2'-[(2,2',4,4',6,6'-hexaiodo-1,1-biphényl)-3,3'-diylbis(oxy)]bisacétique ;
- le 1,1'-[(2,2',4,4',6,6'-hexaiodo-1,1'-biphényl)-3,3'-diylbis[oxy(1-oxo-2,1-éthanediyl)méthylimino]]bis[1-désoxy-D-glucitol] ;
- le 2,2'-[(2,2',4,4',6,6'-hexaiodo-1,1'-biphényl)-3,3'-diylbis(oxy)]bis[N-(2,3-dihydroxypropyl)acétamide] ;
- le 2,2'-[(2,2',4,4',6,6'-hexaiodo-1,1'-biphényl)-3,3'-diylbis(oxy)]bis[N-méthylacétamide] ;
- l'acide 2,2'-[(2,2',4,4',6,6'-hexaiodo-1,1'-biphényl)-3,3'-diylbis(oxy)]bisacétique ;
- le 2,2'-[(2,2',4,4',6,6'-hexaiodo-1,1'-biphényl)-3-3'-diylbis(oxy)]acétamide ;
- le 2,2'-[[2,2',4,4',6,6'-hexaiodo-1,1'-biphényl]-3,3'-diylbis(oxy)]bis[N-[2-(2-hydroxyéthoxy)éthyl]-acétamide] ;
- le 2[3-[3-[(hydroxyacétyl)[2-(2-hydroxyéthoxy)éthyl]-amino]-2,4,6-triiodophényl]-2,4,6-triiodophénoxy]-N-[2-(2-hydroxyéthoxy)éthyl]acétamide ;

4. Composition de milieu de contraste utile pour le diagnostic aux rayons X du corps humain et animal, comprenant au moins l'un des composés selon la revendication 1 ou 3 ou les sels de ceux-ci.

5. Utilisation d'au moins l'un des composés selon les revendications 1 à 3 pour la préparation de formulations de diagnostics aux rayons X.

6. Utilisation de formulations selon la revendication 5, pour obtenir des images d'organes ou de tissus d'un corps humain et animal dans l'imagerie diagnostique aux rayons X.
